(19) Europäisches Patentamt
European Patent Office
Office européen des brevets

(11) Veröffentlichungsnummer: **0 069 883**
**B1**

# (12) EUROPÄISCHE PATENTSCHRIFT

(45) Veröffentlichungstag der Patentschrift: **29.08.84**

(51) Int. Cl.³: **C 07 C 123/02**

(21) Anmeldenummer: **82105416.0**

(22) Anmeldetag: **21.06.82**

(54) **Verfahren zur Herstellung von Hydrazidinen.**

(30) Priorität: **03.07.81 DE 3126388**

(43) Veröffentlichungstag der Anmeldung:
**19.01.83 Patentblatt 83/3**

(45) Bekanntmachung des Hinweises auf die Patenterteilung:
**29.08.84 Patentblatt 84/35**

(84) Benannte Vertragsstaaten:
**AT BE CH DE FR GB IT LI NL**

(56) Entgegenhaltungen:
**JOURNAL OF HETEROCYCLIC CHEMISTRY, Band 12, Nr. 6, Dezember 1975, Seiten 1143-1153, S.A. LANG et al.: "Novel synthesis of unsymmetrically substituted s-tetratzines"**
**JUSTUS LIEBIGS ANNALEN DER CHEMIE, Band 749, 1971, Verlag Chemie GmbH, Seiten 16-17, Weinheim, DE. H. NEUNHOEFFER et al.: "Synthese von Amidrazonen aus Amidinen"**
**JUSTUS LIEBIGS ANNALEN DER CHEMIE, Heft 1, 1975, Seiten 1120-1123, Verlag Chemie GmbH, Weinheim, DE. H. NEUNHOEFFER et al.: "Synthese N-unsubstituierter Hydrazidine"**

(73) Patentinhaber: **BAYER AG, Konzernverwaltung RP Patentabteilung, D-5090 Leverkusen 1 Bayerwerk (DE)**

(72) Erfinder: **Bonse, Gerhard, Dr., Wolfskaul 3, D-5000 Köln 80 (DE)**
Erfinder: **Schmidt, Thomas, Dr., Ginsterweg 9, D-5657 Haan (DE)**

**EP 0 069 883 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues vorteilhaftes, von Amidinen ausgehendes Verfahren zur Herstellung von weitgehend bekannten Hydrazidinen.

Es ist bereits bekannt geworden, daß man bestimmte Hydrazidine erhält, wenn man zunächst in einer ersten Stufe das Amidiniumsalz einer Carbonsäure mit Hydrazin in ein Amidrazonsalz überführt; nach dessen Isolierung wird in einer zweiten Stufe, nach Umsetzung mit wasserfreiem Hydrazin in wasserfreiem Lösungsmittel, die Reaktionsmischung bei vermindertem Druck (ca. 480 mbar) einige Zeit auf 40°C erwärmt und nach Entfernen des Lösungsmittels das entsprechende Hydrazidinsalz erhalten (vgl. Liebigs Ann. Chem. 749, S. 16—23 (1971) und Liebigs Ann. Chem. 1975, S. 1120—1123).

Acethydrazidinhydrochlorid kann so ausgehend von Acetamidinhydrochlorid in einer Gesamtausbeute von 83% der Theorie erhalten werden.

Dieses zweistufige Verfahren hat jedoch den Nachteil, daß neben einer Zwischenisolierung des Amidrazons weiterhin mit wasserfreiem Hydrazin in wasserfreiem Lösungsmittel umgesetzt werden muß. Dies bedeutet einen erheblichen technischen Aufwand; das Arbeiten mit wasserfreiem Hydrazin stellt darüberhinaus bei der technischen Synthese ein Sicherheitsrisiko dar.

Es wurde nun überraschend gefunden, daß man die weitgehend bekannten Hydrazidine der Formel

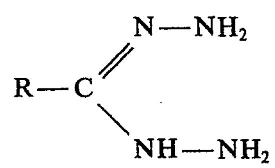

(I)

in welcher

R für gegebenenfalls substituiertes Alkyl, gegebenenfalls substituiertes Cycloalkyl, gegebenenfalls substituiertes Aryl oder gegebenenfalls substituiertes Aralkyl steht,

bzw. deren Säureadditionssalze, ausgehend von entsprechenden Amidinen in sehr guten Ausbeuten und in sehr reiner Form durch eine neue einstufige Verfahrensweise herstellen kann, indem man Amidine von Carbonsäuren der Formel

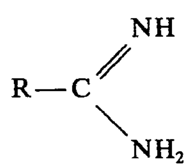

(II)

in welcher

R die oben angegebene Bedeutung hat,

oder deren Säureadditionssalze mit Hydrazinhydrat ($NH_2—NH_2 \times H_2O$) in technischen (wasserhaltigen) Lösungsmitteln bei vermindertem Druck und bei Temperaturen zwischen —80°C und +100°C umsetzt.

Das erfindungsgemäße Verfahren weist eine Reihe von Vorteilen auf. Neben deutlich höheren Ausbeuten ist es besonders zweckmäßig, die Gesamtreaktion als »Eintopfverfahren« durchzuführen. Weiterhin können anstelle von wasserfreiem Hydrazin und absoluten Lösungsmitteln Hydrazinhydrat und technische, d. h. wasserhaltige Lösungsmittel verwendet werden.

Die nach dem erfindungsgemäßen Verfahren herstellbaren Hydrazidine sind durch die Formel (I) allgemein definiert. In dieser Formel steht R vorzugsweise für gegebenenfalls substituiertes geradkettiges oder verzweigtes Alkyl mit 1—12 C-Atomen, gegebenenfalls substituiertes Cycloalkyl mit 3—6 C-Atomen, gegebenenfalls substituiertes Aryl mit 6 oder 10 C-Atomen und für gegebenenfalls substituiertes Aralkyl mit 6 oder 10 C-Atomen im Arylteil und 1—4 C-Atomen im Alkylteil.

Die vorgenannten Reste können grundsätzlich durch solche Gruppen substituiert sein, die unter den Reaktionsbedingungen nicht mit Hydrazinhydrat reagieren.

Beispielsweise können die Alkylreste durch Halogen (insbesondere Chlor oder Fluor) oder durch Alkoxy oder Alkylthio mit jeweils 1—4 C-Atomen substituiert sein.

Die Cycloalkylreste können beispielsweise durch Halogen (insbesondere Chlor oder Fluor), durch Alkyl, Alkoxy oder Alkylthio mit jeweils 1—4 C-Atomen oder durch (gegebenenfalls selbst substituiertes) Phenyl substituiert sein.

Die Aryl- und Aralkylreste können beispielsweise durch Halogen (vorzugsweise Fluor, Chlor oder Brom), durch Nitro, durch Alkyl, Alkoxy oder Alkylthio mit jeweils 1—4 C-Atomen oder durch Halogenalkyl, Halogenalkoxy oder Halogenalkylthio mit jeweils 1—4 C-Atomen und bis zu 5 gleichen oder

verschiedenen Halogenatomen (insbesondere Fluor- und Chloratomen) substituiert sein.

Besonders bevorzugt sind diejenigen Verbindungen der Formel (I), in denen R für Methyl, Chlormethyl, Ethyl, n-Propyl, Isopropyl, n-Butyl, sek.-Butyl, tert.-Butyl, Cyclopropyl, Cyclobutyl, Cyclopentyl, Cyclohexyl, Phenyl oder Benzyl steht.

Verwendet man beispielsweise Acetamidinhydrochlorid und Hydrazinhydrat als Ausgangsstoffe, so kann der Reaktionsablauf durch das folgende zusammenfassende Formelschema wiedergegeben werden:

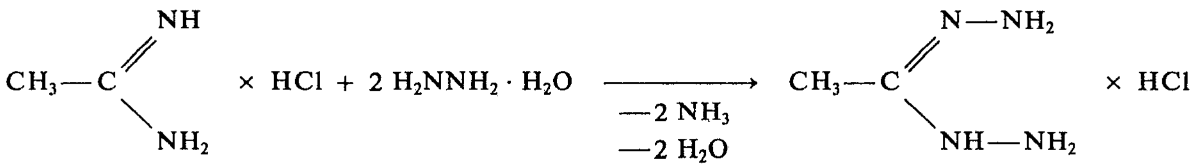


Die bei der Durchführung des erfindungsgemäßen Verfahrens als Ausgangsstoffe benötigten Amidine sind durch die Formel (II) allgemein definiert. In dieser Formel steht R vorzugsweise für diejenigen Reste, die bereits im Zusammenhang mit der Beschreibung der erfindungsgemäß herstellbaren Stoffe der Formel (I) vorzugsweise für diesen Substituenten genannt wurden.

Die Amidine der Formel (II) sowie ihre Säureadditionssalze sind allgemein bekannte Verbindungen der organischen Chemie, bzw. können sie nach bekannten Verfahren hergestellt werden (vgl. Org. Synthesis Coll. Vol. I, S. 5 (1951); Beilstein Bd. 2, S. 185; Bd. 2/III, S. 452; Bd. 2/III, S. 478; Bd. 9, S. 280).

Als Beispiele seien genannt: Acetamidin, Chloracetamidin, Trichloracetamidin, Propionamidin, Butyramidin, Isobutyramidin, Valeramidin, Benzamidin sowie deren Hydrochloride.

Als Verdünnungsmittel kommen für die erfindungsgemäße Umsetzung inerte organische Lösungsmittel in Frage, die durchaus in Form technischer Lösungsmittel bis zu 20% Wasser enthalten können.

Hierzu gehören vorzugsweise Alkohole, wie Methanol, Ethanol, n-Propanol, Isopropanol, n-Butanol, Isobutanol, tert-Butanol, Glycol, Glycerin, Amylalkohol, Cyclohexanol, 2-Methyl-4-pentanol, Methylglycol, n-Hexanol, Isohexylalkohol, Isoheptylalkohol, n-Heptanol, Nonylalkohol, Dodecylalkohol oder Methylcyclohexanol; Ether, wie Tetrahydrofuran, Dioxan oder Ethylenglycolmonomethylether; Amide, wie Dimethylformamid, Diethylformamid, Dimethylacetamid oder N-Methylpyrrolidon; Kohlenwasserstoffe, wie Benzol oder Toluol; sowie halogenierte Kohlenwasserstoffe, wie Methylenchlorid, Chloroform, Tetrachlorkohlenstoff, 1,2-Dichlorethan, 1,1,2-Trichlorethan, Trichlorethylen sowie Chlorbenzol. Es können auch entsprechende Lösungsmittelgemische verwendet werden.

Die Umsetzung wird bei vermindertem Druck durchgeführt, um das bei der Reaktion freigesetzte Ammoniak aus dem Reaktionsgemisch zu entfernen. Es ist zweckmäßig, im Druckbereich von etwa 0,1 bis etwa 800 mbar zu arbeiten; bevorzugt wird bei Drucken zwischen 50 und 300 mbar gearbeitet.

Die Reaktionstemperaturen können in einem größeren Bereich variiert werden und richten sich nach dem jeweils angewandten Druck. Die Umsetzung kann, wie oben angegeben, im Temperaturbereich von etwa −80° bis +100° C durchgeführt werden; bevorzugt arbeitet man zwischen −30° und +50° C. Das Verfahren kann kontinuierlich oder diskontinuierlich durchgeführt werden.

Bei der Durchführung des erfindungsgemäßen Verfahrens setzt man im allgemeinen auf 1 Mol eines Amidins der Formel (II) oder eines entsprechenden Amidin-Säureadditionssalzes 2 bis 2,5 Mol, vorzugsweise 2 bis 2,3 Mol Hydrazinhydrat ein.

Die Isolierung der Verfahrensprodukte der Formel (I) erfolgt in üblicher Weise durch Entfernen des Lösungsmittels. Die nach dem erfindungsgemäßen Verfahren in hoher Reinheit und Ausbeute anfallenden Hydrazidine bzw. Hydrazidin-Säureadditionssalze können vorzugsweise ohne Isolierung als Lösung oder Suspension zu Folgeprodukten umgesetzt werden.

Hydrazidine sind wertvolle Synthesebausteine für zahlreiche Heterocyclen und hier insbesondere für herbizid wirksame as-Triazinone (vgl. Liebigs Ann. Chem. 1976, S. 2206—2221; ibid. 1975, S. 1120—1123; Chem. Ber. 108, S. 3509—3517 (1975); Chem. Ber. 112, S. 1981—1990 (1979); DE-OS 22 24 161; DE-OS 25 56 835; DE-OS 31 02 318).

Die nachfolgenden Beispiele sollen die Erfindung näher erläutern, aber nicht einschränken.

## Herstellungsbeispiele

### Beispiel 1

Acethydrazidinhydrochlorid

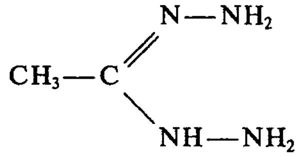

× HCl

In einem 1-Ltr.-Dreihalskolben wird eine Suspension von 30 g (0,3 Mol) Acetamidinhydrochlorid (95%ig) in 200 ml Ethanol auf 5° C gekühlt und ein verminderter Druck von 130 mbar eingestellt. Unter starkem Rühren werden nun 31,4 g (0,62 Mol) Hydrazinhydrat zugetropft und weitere zwei Stunden bei 5° C nachgerührt. Anschließend wird Ethanol im Vakuum abgezogen und man erhält nach dem Trocknen 37,1 g Acethydrazidinhydrochlorid als farblose Kristalle, Schmelzpunkt 154° C, vom Gehalt 99% (polarographisch ermittelt), was einer Ausbeute von 95% der Theorie entspricht.

Nach der im Beispiel zur Herstellung von Acethydrazidin beschriebenen Verfahrensweise können auch die in der folgenden Tabelle aufgeführten Verbindungen der Formel

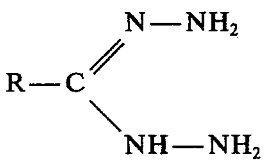

× HCl (Ia)

hergestellt werden:

Tabelle

| Beispiel Nr. | R | Ausbeute (% der Theorie) | Schmelzpunkt (° C) |
|---|---|---|---|
| 2 | $C_2H_5$ | 85 | 109—112 |
| 3 | n-$C_3H_7$ | 81 | 105—115 |
| 4 | iso-$C_3H_7$ | 69 | 126—128 |
| 5 | $ClCH_2$ | 75 | 135—138 |
| 6 | $Cl_3C$ | 60 | 112—115 |

**Patentansprüche**

1. Verfahren zur Herstellung von Hydrazidinen der Formel

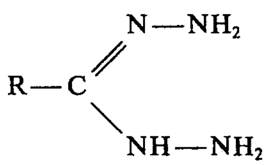

(I)

in welcher

R für gegebenenfalls substituiertes Alkyl, gegebenenfalls substituiertes Cycloalkyl, gegebenenfalls substituiertes Aryl oder gegebenenfalls substituiertes Aralkyl steht,

oder von deren Säureadditionssalzen, ausgehend von den entsprechenden Amidinen, dadurch gekennzeichnet, daß man Amidine der Formel

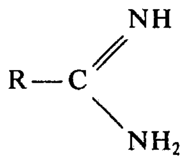
(II)

in welcher

R die oben angegebene Bedeutung hat,

oder deren Säureadditionssalze mit Hydrazinhydrat ($NH_2—NH_2 \times H_2O$) in technischen (wasserhaltigen) Lösungsmitteln bei vermindertem Druck und bei Temperaturen zwischen —80° und +100°C umsetzt.

2. Verfahren nach Anspruch 1, dadurch gekennzeichnet, daß man im Druckbereich von 0,1—800 mbar arbeitet.

3. Verfahren nach Anspruch 1, dadurch gekennzeichnet, daß man bei Drücken zwischen 50 und 300 mbar arbeitet.

4. Verfahren nach Anspruch 1, dadurch gekennzeichnet, daß man bei Temperaturen zwischen —30° und +50°C arbeitet.

5. Verfahren nach Anspruch 1, dadurch gekennzeichnet, daß man auf 1 Mol eines Amidins der Formel (II) oder eines entsprechenden Amidin-Säureadditionssalzes 2—2,5 Mol, vorzugsweise 2—2,3 Mol Hydrazinhydrat einsetzt.

6. Verfahren nach Anspruch 1, dadurch gekennzeichnet, daß man als Ausgangsstoffe Acetamidinhydrochlorid und Hydrazinhydrat einsetzt.

**Claims**

1. Process for the preparation of hydrazidines of the formula

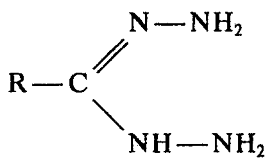
(I)

in which

R represents optionally substituted alkyl, optionally substituted cycloalkyl, optionally substituted aryl or optionally substituted aralkyl,

or of their acid addition salts, starting from the corresponding amidines, characterised in that amidines of the formula

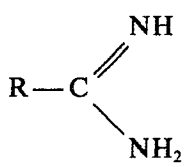
(II)

in which

R has the meaning given above,

or their acid addition salts, are reacted with hydrazine hydrate ($NH_2—NH_2 \times H_2O$) in technical solvents (containing water) under reduced pressure and at temperatures between —80° and +100°C.

2. Process according to Claim 1, characterised in that a pressure in the range from 0.1 to 800 mbar is employed.

3. Process according to Claim 1, characterised in that pressures between 50 and 300 mbar are employed.

4. Process according to Claim 1, characterised in that temperatures between —30° and +50°C are employed.

5. Process according to Claim 1, characterised in that 2—2.5 mol, preferably 2—2.3 mol, of hydrazine hydrate are employed for 1 mol of an amidine of the formula (II) or a corresponding acid addition salt of an amidine.

6. Process according to Claim 1, characterised in that the starting materials employed are acetamidine hydrochloride and hydrazine hydrate.

**Revendications**

1. Procédé de préparation d'hydrazidines de formule

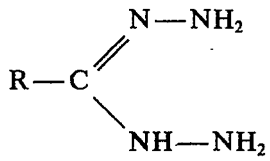

(I)

dans laquelle

R représente un alcoyle éventuellement substitué, un cycloalcoyle éventuellement substitué, un aryle éventuellement substitué ou un aralcoyle éventuellement substitué,

ou de leurs sels d'addition d'acides, au départ des amidines correspondantes, caractérisé en ce qu'on fait réagir des amidines de formule

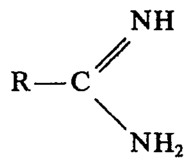

(II)

dans laquelle

R a la signification indiquée plus haut

ou leurs sels d'addition d'acides, avec de l'hydrate d'hydrazine ($NH_2-NH_2 \times H_2O$) dans des solvants techniques (renfermant de l'eau) sous pression réduite et à des températures entre $-80°C$ et $+100°C$.

2. Procédé selon la revendication 1, caractérisé en ce qu'on opère dans l'intervalle de pression de 0,1—800 mbars.

3. Procédé selon la revendication 1, caractérisé en ce qu'on opère sous des pressions entre 50 et 300 mbars.

4. Procédé selon la revendication 1, caractérisé en ce qu'on opère à des températures entre $-30°C$ et $+50°C$.

5. Procédé selon la revendication 1, caractérisé en ce qu'on utilise pour 1 mole d'une amidine de formule (II) ou d'un sel d'addition d'amidine correspondant 2—2,5 moles, de préférence 2—2,3 moles d'hydrate d'hydrazine.

6. Procédé selon la revendication 1, caractérisé en ce qu'on utilise comme matières premières du chlorhydrate d'acétamidine et de l'hydrate d'hydrazine.